# EUROPEAN PATENT SPECIFICATION

(11) **EP 0 467 488 B1**
(45) Date of publication and mention of the grant of the patent: **11.10.1995**
(21) Application number: 91202100.3
(22) Date of filing: 17.08.1984
(51) Int. Cl.: A61K 31/135, A61K 9/36

(54) **Composition for the controlled discharge of an active ingredient**
Zusammensetzung zur kontrollierten Abgabe eines Wirkstoffs
Composition à libération contrôlée d'une substance active

(43) Date of publication of application: 22.01.1992
(62) Divisional of application: 84109832.0
(73) Proprietor: THE WELLCOME FOUNDATION LIMITED, London NW1 2BP (GB)
(72) Inventor: Baker, Richard William, Mountain View California 94303 (US); Brooke, James William, Sisters Oregon 97759 (US); Smith, Kelly Lincoln, Bend Oregon 97701 (US)
(74) Representative: Stott, Michael John

(56) References cited:
- GB-A- 2 123 291
- US-A- 2 602 040
- US-A- 4 425 363
- US-A- 4 428 926
- US-A- 4 687 660

## Description

The present invention relates to a novel composition which affords a continuous discharge of an active ingredient in a controlled manner.

U.S. patents 3,845,770 and 3,916,899 relate to a composition or device comprising an active ingredient and a semipermeable membrane surrounding the active ingredient and through which is provided a passageway. In use, the composition is contacted with a fluid which permeates or diffuses through the membrane and dissolves the active ingredient. In this way, an osmotic pressure gradient is established across the membrane with the result that the solution of the active ingredient is discharged or released through the passageway into the ambient fluid. U.S. patents 4,160,452 and 4,200,098 relate to similar compositions in which a passageway or portal is also used for the discharge of the solution.

U.S. patent 4,016,880 relates to a composition or dispenser in which the semipermeable membrane or wall is provided with sites of structural weakness. As a result of the osmotic pressure gradient that builds up in use, the membrane fractures at the sites of weakness thereby forming passageways in situ, through which the solution is discharged.

J.Pharm.Sci., 1983, 72/7, pages 772 to 775 relates to tablets coated with a polyvinyl chloride membrane in which is dispersed particulate water-soluble pore-forming material. In use, an aqueous liquid dissolves the particulate water-soluble pore-forming material to give a highly porous membrane in situ. The aqueous liquid then gains access through the pores so formed to the tablet within the membrane and dissolves it, the resulting solution discharging out of the membrane through the pores.

Bupropion is a known pharmaceutical with antidepressant proprieties (see US-A-3819706 and US-A-3885046). As used herein, the term "active ingredient" means bupropion hydrochloride.

The present invention provides a pharmaceutical composition adapted for oral administration comprising bupropion hydrochloride as active ingredient formulated to afford a continuous release of active ingredient in a controlled manner.

The pharmaceutical composition is in the form of tablets or pills or capsules containing prill seed compositions. The composition may comprise a formulation containing the active ingredient, a semipermeable membrane surrounding the formulation, and particulate water-soluble pore-forming material dispersed within the membrane, whereby, in us in an aqueous environment, the pore-forming material is dissolved forming pores in the semipermeable membrane, the active ingredient is taken up in solution thus creating an osmotic pressure gradient across the membrane between the solution and the aqueous environment, and water from the aqueous environment is diffused through the semipermeable membrane into contact with the active ingredient concurrently while a solution of the active ingredient is discharged through the pores of the membrane into the aqueous environment.

Examples of the formulation containing the active ingredient include a compressed form of the active ingredient optionally in admixture with one or more excipients, and a prill seed, as defined hereinafter, having a coating of a water-permeable polymer, in which seed or coating or both the active ingredient is dispersed. In the former case, the compressed form is a tablet or a pill and preferred examples of an excipient include an osmotic enhancing agent and standard formulating excipients, such as a filler and a binding agent. Such tablet or pill forms are prepared in accordance with standard techniques known in the art of pharmacy.

As used herein, the prill seed of use with the polymer-coated prill seeds described above are solid particles, the largest dimension of which is from 0.1 to 4.0mm, usually from 0.2 to 0.3mm. They are normally substantially spherical in shape but may be ovoidal or even of irregular shape, and they are generally used in capsule dosage forms. Preferred examples of prill seeds include those produced from sugar such as sucrose and mannitol, starch, salt such as sodium chloride, and wax.

The preparation of such prill seeds may be carried out in accordance with standard techniques known in the art of pharmacy. Alternatively, a number of different types of prill seeds are commercially available from, for example, Ingredient Technology Corporation, Pennsauken, New Jersey, U.S.A.

The water-permeable polymer coating of use with the polymer-coated prill seeds described above may be the same polymer material as is described hereinafter in relation to the semipermeable membrane but is, preferably, a polymeric material having a higher permeability to water. Examples of such latter material include cellulose acetate. It is also preferred that the active ingredient is dispersed within the polymeric material rather than within the prill seed. In such circumstances, from 0.5 to 5.0 parts of active ingredient on a weight basis are normally used for every one part of polymer.

The polymer-coated prill seeds may be prepared by standard techniques known in the art of pharmacy. For example, the prill seeds may be coated with a solution of the polymer optionally containing (also in solution but, if not, then in suspension) the active ingredient. Such coating is usually carried out on a fluidized bed coater in which the coating solution is sprayed into the suspension air stream thereby coating the prill seeds.

As used herein, an osmotic enhancing agent is a substance having a high molar water solubility and which is capable of achieving, in use of the composition in an aqueous environment, an increase in the osmotic pressure within the composition relative to the osmotic pressure of the aqueous environment. Examples of an osmotic enhancing agent include sugar such as sucrose, lactose, fructose and mannitol, and salt such as sodium chloride, potassium chloride and sodium carbonate.

As used herein, a semipermeable membrane is a membrane that is permeable to water but not permeable to the active ingredient or any osmotic enhancing agent that may be present. The membrane should not be detrimental to the active ingredient and should be suitable for the use to which the composition is intended to be put. The thickness of the membrane is generally from 10 to 500µm, preferably from 25 to 50µm. The membrane may be made of any material that is suitable for use in reverse osmosis or has application in dialysis. General examples of such material include cellulose esters such as mono-, di- and tri-acylates including mixed esters, cellulose ethers such as ethyl cellulose, nylons, polycarbonates, poly(dialkylsiloxanes), poly(methacrylic acid) esters, poly(acrylic acid) esters, poly(phenylene oxides), poly(vinyl alcohols), aromatic nitrogen-containing polymers, polymeric epoxides and regenerated cellulose. Specific examples include cellulose acetate, cellulose diacetate, cellulose triacetate, cellulose propionate, cellulose acetate butyrate, cellulose acetate propionate, cellulose tripropionate, ethyl cellulose and nylon 6.

The semipermeable membrane may optionally contain one or more additives, such as a plasticizer and a water permeability-modifying agent. The plasticizer is, preferably, a non-migrating plasticizer, general examples of which include esters such as a phthalate, phosphate, citrate, adiphate, tartrate, sebacate, succinate, glycolate, glycerolate, benzoate and myristate esters and sulfonamides. Specific examples include dimethyl phthalate, dipropyl phthalate, di-(2-ethylhexyl)phthalate, tributyl phosphate, triacetyl phospate, and tributyl citrate.

As used herein, a water permeability-modifying agent is a material which is capable of enhancing the permeability of water through the semipermeable membrane. General examples of such a material include the poly(alkylene glycols), esters and polyesters of poly(alkylene glycols), polyhydric alcohols and esters and polyesters of polyhydric alcohols. Specific examples include poly(ethylene glycols) 300, 400, 600, 1500 and 1540, poly(propylene glycol), 1,3-butyleneglycol, glycerine, ethylene glycol dipropionate and ethylene glycol butyrate.

The particulate water-soluble pore-forming material of use in the composition of the present invention, preferably, has a maximum particle size not exceeding 500µm in its longest dimension and has an average particle size from 5 to 100µm. The material is, preferably, also insoluble in the organic solvent in which the polymeric material is dissolved for forming the semipermeable membrane as described hereinafter. Examples of water soluble pore-forming material include water-soluble sugars such as lactose, sucrose, sorbitol and mannitol, and salts such as sodium carbonate, sodium chloride, calcium chloride, potassium chloride and sodium sulphate.

The composition described above may be prepared by a process which comprises coating a formulation containing the active ingredient with a coating mix containing in an organic solvent a solution of a material for forming a semipermeable membrane that surrounds the formulation and a suspension of particulate water-soluble pore-forming material for dispersion within the membrane, and drying the coated formulation.

The coating operation may be carried out by spraying the coating mix on to the formulation in, for example, a rotating pan coater or fluidized bed coater. The drying operation is carried out conventionally.

As mentioned previously, it is preferred that the particulate water-soluble pore-forming material is insoluble in the organic solvent. If, however, it is not, then a suspension of the material may be obtained by suspending it in a solvent in which it is insoluble, and then coating the formulation separately but simultaneously with the solution and the suspension.

Examples of an organic solvent include acetone.

The present invention will now be further described with reference to drawings and examples, neither of which should be construed as limiting the invention in any way.

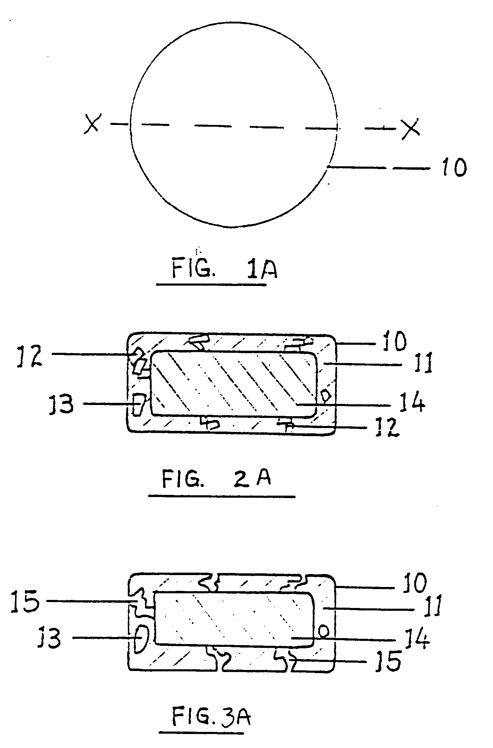

In the drawings which are not drawn to scale, Figures 1A and 1B are plan views of a composition of the invention in which the formulation is a tablet (1A) or a polymer-coated prill seed (1B);
Figures 2A and 2B are sectional views taken along the line X-X in a horizontal direction and illustrating the structures of the compositions before their use in an aqueous environment;
Figures 3A and 3B are the same views as depicted in Figures 2A and 2B respectively but illustrating the structures of the compostions after the pore-forming material has been dissolved.

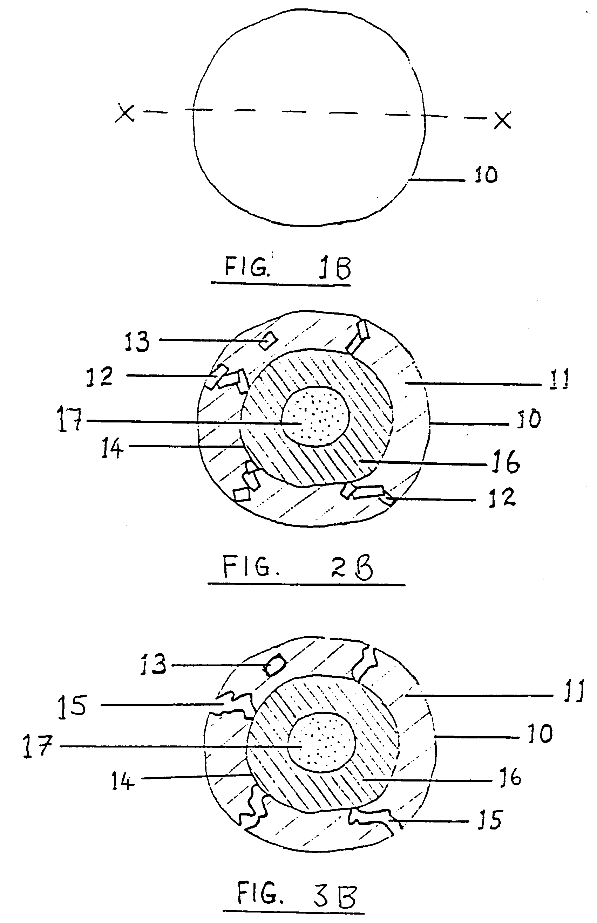

Referring to the Figures, there are shown compositions 10 comprising a tablet 14 containing the active ingredient (in Figures 1A, 2A and 3A), or a Polymer-coated prill seed 14, the polymer-coating 16 containing the active ingredient (in Figures 1B, 2B and 3B), a semipermeable membrane 11, and particulate water-soluble pore-forming material 12 and 13 dispersed within the membrane 11. In the majority of places, the pore-forming material 12 is aggregated together across the thickness of the membrane 11 whilst in a few places the material 13 is not so aggregated.

In use of the composition 10 in an aqueous environment (not shown), the pore-forming material 12 that is aggregated together across the thickness of the membrane 11 is dissolved in water from the aqueous environment thus forming pores 15 in the membrane 11. Some of the active ingredient within the tablet 14 (in Figures 1A, 2A and 3A) or within the polymer coating (in Figures 1B, 2B and 3B) in the immediate vicinity of the pores 15 then comes into contact with water and is taken up in solution thus setting up an osmotic pressure gradient across the membrane 11 between the aqueous solution of the active ingredient and the aqueous environment. The effect of the pressure gradient so created is that water from the aqueous environment diffuses or permeates through the membrane 11 into contact with the active ingredient while an aqueous solution of the active ingredient is discharged through the pores 15 of the membrane 11 into the aqueous environment and this process continues until the concentration of the solution of the active ingredient within the membrane 11 is substantially the same as that outside the membrane 11 in the aqueous environment at which point there is no longer any osmotic pressure gradient between the two solutions, the bulk of the active ingredient having been discharged as an aqueous solution from the composition 10 into the aqueous environment.

### EXAMPLE 1

Tablets containing 100mg bupropion hydrochloride and 500mg lactose were prepared using a conventional tablet press. Fifty tablets were placed in a miniature pan coater. A polymer solution was prepared by dissolving cellulose acetate (CA 383-40 from Eastman Chemical Products, Inc., Kingsport, Tenn.) and poly(ethylene glycol) (Polyglycol E-400 from Dow Chemical Co., Midland, Mich.) in acetone and adding impalpable lactose (particle size: 2-20µm) to give a mixture containing cellulose acetate: poly(ethylene glycol): lactose in the weight % ratio of 40:40:20 and a total solids content of 50g/L. The polymer mixture was sprayed onto the tablets in the pan coater to give membrane-coated tablets, the membrane coating weighing 27mg each when dried.

Drug release rates were determined for the tablet compositions by placing them in simulated gastric buffer (pH 1.5) at 37°C and periodically measuring the bupropion hydrochloricde concentration in the buffer. After 2 hr., about 45% of the bupropion hydrochloride was released; after 4 hr., about 70%; and after 6 hr., about 90%.

### EXAMPLE 2

Tablets containing 100mg bupropion hydrochloride and 500mg lactose were prepared using a conventional tablet press. Fifty tablets were placed in a miniature pan coater. A polymer solution was prepared by dissolving cellulose acetate (CA 383-40) and poly(ethylene glycol) (Polyglycol E-400) in acetone and adding impalpable lactose to give a mixture containing cellulose acetate: poly(ethylene glycol): lactose in the weight % ratio of 67:13:20 and a total solids content of 50g/L. The polymer mixture was sprayed onto the tablets in the pan coater to five membrane-coated tablets, the membrane coating weighing 35mg each when dried.

Drug release rates were determined for the table compositions by placing them in simulated gastric buffer (pH 1.5) at 37°C and periodically measuring the bupropion hydrochloride concentration of the buffer. After 2 hr., about 10% of the bupropion hydrochloride was released; after 4hr., about 25%; after 6 hr., about 40%; and after 8 hr., about 55%.

## Claims

1. A pharmaceutical composition adapted for oral administration comprising bupropion hydrochloride as active ingredient characterised in that the composition is a tablet, pill or capsule formulated to afford a continuous release of active ingredient in a controlled manner.

2. A pharmaceutical composition as claimed in claim 1 characterised in that up to about 45% of the active ingredient is released over a period of 2 hours in simulated gastric buffer.

3. A pharmaceutical composition as claimed in claim 1 or 2 characterised in that up to about 70% of the active ingredient is released over a period of 4 hours in simulated gastric buffer.

4. A pharmaceutical composition as claimed in any of claims 1 to 3 characterised in that up to about 90% of the active ingredient is released over a period of 6 hours in simulated gastric buffer.

5. A pharmaceutical composition as claimed in claim 1 characterised in that from about 10% to about 45% of the active ingredient is released over a period of 2 hours in simulated gastric buffer.

6. A pharmaceutical composition as claimed in claim 1 or 5 characterised in that from about 25% to about 70% of the active ingredient is released over a period of 4 hours in simulated gastric buffer.

7. A pharmaceutical composition as claimed in any of claims 1, 5 or 6 characterised in that from about 40% to about 90% of the active ingredient is released over a period of 6 hours in simulated gastric buffer.

8. A pharmaceutical composition as claimed in any of claims 1 to 7 characterised in that the unit dose of bupropion hydrochloride is 100 mg.

## Patentansprüche

1. Pharmazeutische Zubereitung für die orale Verabreichung enthaltend Bupropion-Hydrochlorid als Wirkstoff, **dadurch gekennzeichnet,** daß die Zubereitung in Form einer Tablette, Pille oder Kapsel vorliegt, die so formuliert ist, daß sie eine kontinuierliche Freisetzung des Wirkstoffs in gesteuerter Weise ergibt.

2. Pharmazeutische Zubereitung nach Anspruch 1, **dadurch gekennzeichnet,** daß bis zu etwa 45% des Wirkstoffs im Verlaufe von 2 Stunden in einem simulierten Magensaftpuffer freigesetzt werden.

3. Pharmazeutische Zubereitung nach Anspruch 1 oder 2, **dadurch gekennzeichnet,** daß bis zu etwa 70% des Wirkstoffs im Verlaufe von 4 Stunden in einem simulierten Magensaftpuffer freigesetzt werden.

4. Pharmazeutische Zubereitung nach einem der Ansprüche 1 bis 3, **dadurch gekennzeichnet,** daß bis zu etwa 90% des Wirkstoffs im Verlaufe von 6 Stunden in einem simulierten Magensaftpuffer freigesetzt werden.

5. Pharmazeutische Zubereitung nach Anspruch 1, **dadurch gekennzeichnet,** daß etwa 10% bis etwa 45% des Wirkstoffs im Verlaufe von 2 Stunden in einem simulierten Magensaftpuffer freigesetzt werden.

6. Pharmazeutische Zubereitung nach Anspruch 1 oder 5, **dadurch gekennzeichnet,** daß etwa 25% bis etwa 70% des Wirkstoffs im Verlaufe von 4 Stunden in einem simulierten Magensaftpuffer freigesetzt werden.

7. Pharmazeutische Zubereitung nach einem der Ansprüche 1, 5 oder 6, **dadurch gekennzeichnet,** daß etwa 40% bis etwa 90% des Wirkstoffs im Verlaufe von 6 Stunden in einem simulierten Magensaftpuffer freigesetzt werden.

8. Pharmazeutische Zubereitung nach einem der Ansprüche 1 bis 7, **dadurch gekennzeichnet,** daß die Einheitsdosis des Bupropion-Hydrochlorids 100 mg beträgt.

## Revendications

1. Composition pharmaceutique adaptée a une administration orale comprenant du chlorhydrate de bupropione comme ingrédient actif, caracterisée en ce que la composition est un comprimé, une pilule ou une capsule formulé de façon à permettre une libération continue d'ingrédient actif d'une manière contrôlée.

2. Composition pharmaceutique telle que revendiquée dans la revendication 1, caractérisée en ce que jusqu'à environ 45 % de l'ingrédient actif est libéré en une période de 2 heures dans du tampon gastrique simulé.

3. Composition pharmaceutique telle que revendiquée dans la revendication 1 ou 2, caractérisée en ce que jusqu'à environ 70 % de l'ingrédient actif est libéré en une période de 4 heures dans du tampon gastrique simulé.

4. Composition pharmaceutique telle que revendiquée dans l'une quelconque des revendications 1 à 3, caractérisée en ce que jusqu'à environ 90 % de l'ingrédient actif est libéré en une période de 6 heures dans du tampon gastrique simulé.

5. Composition pharmaceutique, telle que revendiquée dans la revendication 1, caractérisée en ce que environ 10 % à environ 45 % de l'ingrédient actif est libéré en une période de 2 heures dans du tampon gastrique simulé.

6. Composition pharmaceutique telle que revendiquée dans la revendication 1 ou 5, caractérisée en ce que environ 25 % à environ 70 % de l'ingrédient actif est libéré en une période de 4 heures dans du tampon gastrique simulé.

7. Composition pharmaceutique telle que revendiquée dans l'une quelconque des revendications 1, 5 ou 6, caractérisée en ce que environ 40 % à environ 90 % de l'ingrédient actif est libéré en une période de 6 heures dans du tampon gastrique simulé.

8. Composition pharmaceutique telle que revendiquée dans l'une quelconque des revendications 1 à 7, caractérisée en ce que la dose unitaire de chlorhydrate de bupropione est de 100 mg.
